# EUROPEAN PATENT SPECIFICATION

(11) **EP 3 718 116 B1**
(45) Date of publication and mention of the grant of the patent: **10.01.2024**
(21) Application number: 18800985.6
(22) Date of filing: 20.11.2018
(51) Int. Cl.: G16H 10/60, G16H 10/20, G16H 50/70

(54) **APPARATUS FOR PATIENT DATA AVAILABILITY ANALYSIS**
VORRICHTUNG ZUR PATIENTENDATENVERFÜGBARKEITSANALYSE
APPAREIL D'ANALYSE DE LA DISPONIBILITÉ DES DONNÉES DE PATIENTS

(30) Priority: 01.12.2017 WO PCT/CN2017/114205; 26.12.2017 EP 17210579
(43) Date of publication of application: 07.10.2020
(73) Proprietor: Koninklijke Philips N.V., 5656 AG Eindhoven (NL)
(72) Inventor: CHAN, Tak, Ming, 5656 AE Eindhoven (NL); FENG, Jinghan, 5656 AE Eindhoven (NL); TAO, Liang, 5656 AE Eindhoven (NL); CHIAU, Choo, Chiap, 5656 AE Eindhoven (NL)
(74) Representative: Philips Intellectual Property & Standards
(86) International application number: PCT/EP2018/081888
(87) International publication number: WO 2019/105800

(56) References cited:
- WO-A1-2014/201515
- US-A1- 2015 220 868
- US-A1- 2016 378 919

## Description

### FIELD OF THE INVENTION

The present invention relates to an apparatus for patient data availability analysis, to a system for patient data availability analysis, to a method for patient data availability analysis, as well as to a computer program element.

### BACKGROUND OF THE INVENTION

The general background of this invention is the field of identifying patient records. Cardiovascular Information Systems (CVIS) have been available for many years in the healthcare industry. One activity of utilizing information and preferably knowledge from CVIS or Clinical Data repositories (CDR) is to find historical cases that match certain new patient or query criteria so as to provide valuable reference to the decision maker, e.g. the care giver or the physician. There is the trend of data analytics done on collected data from CDR, which provide tremendous valuable data without the heavy dedicated cost and labor of formal clinical studies. For a typical clinical research problem, a number of variables (features) are needed to be extracted from a CDR and there can be multiple ways to obtain these variables from different sources through different rules, yet resulting in different data availability due to noises in the data. CDR data contains noise because clinical practice necessarily balances the treatment of patients, in a timely manner, with the making and recording of absolutely complete and accurate with respect to the patient and the treatment. In addition, due to humans being involved in the processing and recording of data, noise is again introduced due to such incomplete data.

US2016/0019265A1 describes techniques for generating automated advice with respect to consolidating a plurality of sources. In an embodiment, a set of one or more parameters relating to a proposed consolidation for a plurality of consolidation sources is received. In response to receiving the set of one or more parameters, a set of one or more recommendations for consolidating the plurality of consolidation sources is generated and stored on at least one of a volatile or non-volatile computer-readable storage medium. In some embodiments, the set of one or more recommendations may indicate how to improve a performance associated with consolidating the plurality of sources to a set of one or more destinations based on a particular consolidation scenario. The set of one or more recommendations may be displayed during consolidation planning for the plurality of consolidation sources.

WO 2014/201515 A1 discloses systems and processes for processing medical data, e.g. , for determining a likelihood, or risk, of an adverse event or outcome for a person based on machine learning techniques. The outcome may be, for example, a risk of attempting suicide, a probability of cancer survival, a number of re-hospitalisations, etc. This prior art does not disclose determining clean data records as those data records of the available data records that are missing less than a threshold number of values; determining an availability measure comprising the number of clean data records for a given model; and utilizing said availability measures to rank and select a sub-set of models of the plurality of models as top models of data availability.

### SUMMARY OF THE INVENTION

It would be advantageous to have an improved apparatus and associated method for patient data availability analysis, and an improved system for patient data availability analysis, to enable self-service of clinical research data records that includes noisy practice data.

The object of the present invention is solved with the subject matter of the independent claims, wherein further embodiments are incorporated in the dependent claims. It should be noted that the following described aspects and examples of the invention apply also for the apparatus for patient data availability analysis, the system for patient data availability analysis, the method for patient data availability analysis, and for the computer program element.

According to a first aspect, there is provided an apparatus for patient data availability analysis, comprising:
- an input module;
- a data and model analysis module; and
- an optimization module.

The input module is configured to enable a user to specify a plurality of models, each model being used in clinical research and predicting a disease outcome. Each model provides output as a function of model variables. The input module is configured also to enable the user to define at least some model variables for the plurality of models. The input module is configured also to enable the user to specify source variables. A model variable can be derived from one or more source variables. The input module is configured also to enable the user to specify at least one data source. The input module is configured also to receive a plurality of data records from the at least one data source. Each data record comprises at least one attribute. The data and model analysis module is configured to determine a set of available data records for each model from the plurality of data records. The determination for a model comprises utilization of the model variables for that model, the associated source variables and the at least one attribute for the plurality of data records. The data and model analysis module is configured to determine a number of clean data records of the available data records for each model, wherein each of the available data records comprises a plurality of values. The clean data records can then be determined as those data records of the available data records that are missing less than a threshold number of values.
The data and model analysis module is configured also to determine a plurality of availability measures for the corresponding plurality of models, the determination comprising utilization of the determined set of available data records for each model. The availability measure for a model comprises the number of clean data records for that model.
The optimization module is configured to rank and select a sub-set of models of the plurality of models as top models of data availability. The selection comprises utilization of the plurality of availability measures.

In this manner, users can conveniently understand the trade-off between desired input variables and the data size that supports the set of desired variables. They can obtain the optimized availability of data according to their requirements on data characteristics. They can also identify potential data quality issues by variable-availability profiling, and check if the data supports their study hypothesis to be further validated by follow-up data analytics.

To put this another way, data availability is analysed during shortlisting among multiple hypotheses (models), enabling further prediction and modelling steps using that available data and shortlisted models to be more efficient and effective.

Thus, data availability selection at the first level is carried out to determine the best availability among different variable source and derivation choices to simplify and optimize decisions on follow-up analysis, otherwise each model would have non-trivial combinations to be selected and arbitrary choices would introduce challenges of data quality in later modelling steps.

In an example, the input module is configured to enable a user to provide constraints of expected data, and the data and model analysis module is configured to determine for each model of the plurality of models statistics of variables specified in the constraints of expected data provided by the user. The selection of the sub-set of models can then comprise utilization of the determined statistics of variables for the plurality of models.

In an example, the optimization module is configured to output information on whether any data records of the available data records for the top models are outside of the constraints of expected data input by the user.

In other words, the apparatus outputs the information of compromised user expectations (violated constraints), enabling the user to better determine if a model and its associated available data is acceptable.

In an example, the data and model analysis module is configured to determine at least one model performance for at least one model of the plurality of models. The selection of the sub-set of models can then comprise utilization of the at least one model performance.

In an example, the optimization module is configured to determine a best model of the sub-set of models.

In an example, the optimization module is configured to rank the sub-set of models.

In an example, the availability measure for the model comprises the number of clean data records for that model divided by the total number of clean data records.

In an example, the threshold value is a percentage of the number of values, which comprises zero percent.

Thus, clean data records can be those data records that have no missing values, or those data records that have a number of missing values that is below a threshold percentage.

In an example, the availability measure for a model has a value that is weighted by the number of model variables for that model.

In an example, the data and model analysis module is configured to derive at least one model variable for at least one of the plurality of models. The derivation comprises matching at least some of the model variables input by the user with corresponding at least one source variable.

In this way, the apparatus can determine the correct model variables to use for a model on the basis of the information provided by the user.

In an example, the input module is configured to enable the user to input a search query. The user specifying the plurality of models can comprise the input unit identifying at least one model according to the search query.

In other words, the user can explicitly define the models to be used, but can also provide search queries and the input unit retrieves existing models, such as risk models, according to the user search or key words, from knowledge bases in the clinical domain, and thus simplifies the input of model variable and variable derivation rules.

According to a second aspect, there is provided a system for patient data availability analysis, comprising:
- at least one data source;
- an apparatus for patient data availability analysis according to the first aspect and optionally any associated example; and
- an output unit.

The plurality patient records are provided from the at least one data source to the input unit and the output unit is configured to output information relating to the top models of data availability.

According to a third aspect, there is provided a computer-implemented method for patient data availability analysis, comprising:
a) specifying a plurality of models, wherein each model being used in clinical research and predicting a disease outcome, wherein each model provides output as a function of model variables;
b) defining at least some model variables for the plurality of models;
c) specifying source variables, wherein a model variable can be derived from one or more source variables;
d) specifying at least one data source;
e) receiving a plurality of data records from the at least one data source, wherein each data record comprises at least one attribute;
f) determining a set of available data records for each model from the plurality of data records, the determination for a model comprising utilizing the model variables for that model, the associated source variables and the at least one attribute for the plurality of data records; and determining a number of clean data records of the available data records for each model, wherein each of the available data records comprises a plurality of values, and wherein the clean data records are determined as those data records of the available data records that are missing less than a threshold number of values;
g) determining a plurality of availability measures for the corresponding plurality of models, the determination comprising utilizing the determined set of available data records for each model; and wherein the availability measure for a model comprises the number of clean data records for that model; and
h) selecting a sub-set of models of the plurality of models as top models of data availability, wherein the selection comprises utilizing the plurality of availability measures.

Thus an advanced data analytics method can be a directly integrated module in a Cardiovascular Information Systems (CVIS)/ Clinical Data Repository (CDR) to allow users/physician to turn the information stored into knowledge effective and efficiently.

According to another aspect, there is provided a computer program element controlling apparatus and/or system as previously described which, if the computer program element is executed by a processing unit, is adapted to perform the method steps as previously described.

There is also provided a computer readable medium having stored the computer element as previously described.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an apparatus for patient data availability analysis;
Fig. 2 shows a schematic set up of an example of a system for patient data availability analysis;
Fig. 3 shows a method for patient data availability analysis;
Fig. 4 shows a detailed high-level view of a system for patient data availability analysis relating to the example of Acute Kidney Injury (AKI); and
Fig. 5 shows a detailed workflow relating to a system for patient data availability analysis from multiple data sources that include CDR, Laboratory Information System (LIS), and Admission Notes (AN)

### DETAILED DESCRIPTION OF EMBODIMENTS

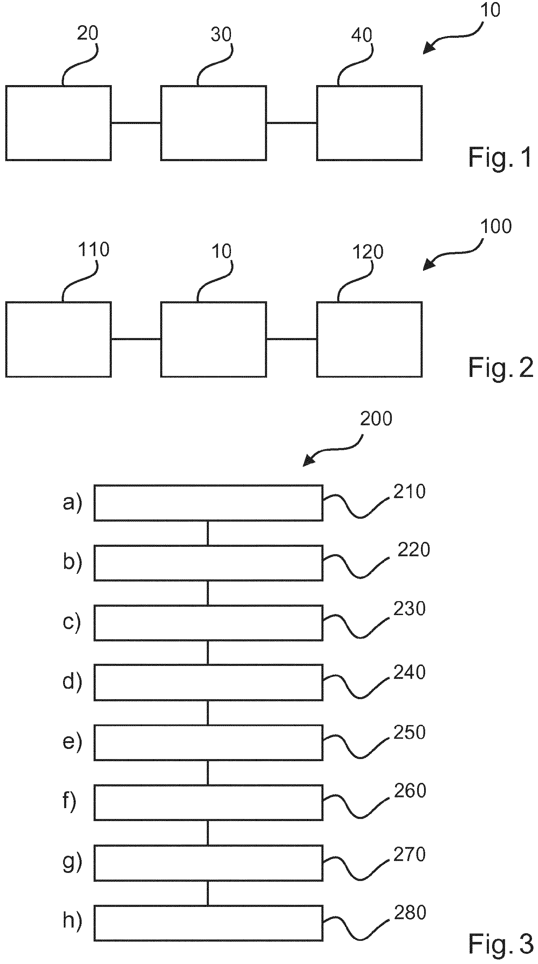

Fig. 1 shows an example of an apparatus 10 for patient data availability analysis. The apparatus 10 comprises an input module 20, a data and model analysis module 30, and an optimization module 40. The input module 20 is configured to enable a user to specify a plurality of models. Each model provides output as a function of model variables. The input module 20 is configured also to enable the user to define at least some model variables for the plurality of models. The input module 20 is configured further to enable the user to specify source variables; a model variable can be derived from one or more source variables. The input module 20 is configured to enable the user to specify at least one data source. The input module 20 is configured also to receive a plurality of data records from the at least one data source; each data record comprises at least one attribute. The data and model analysis module 30 is configured to determine a set of available data records for each model from the plurality of data records, the determination for a model comprises utilization of the model variables for that model, the associated source variables and the at least one attribute for the plurality of data records. The data and model analysis module 30 is configured also to determine a plurality of availability measures for the corresponding plurality of models. The determination of the plurality of availability measures for the corresponding plurality of models comprises utilization of the determined set of available data records for each model. The optimization module 40 is configured to select a sub-set of models of the plurality of models as top models of data availability. The selection comprises utilization of the plurality of availability measures.

In an example, the at least one attribute value comprises one or more of: demographic information, life style information, medical information, care provider information, history and risk factor information, previous visit information, procedure information, etc. of a specific patient. The medical information includes a patient's basic health information, lesion information, device information and follow-up information. The at least one attribute can therefore can include demographic data (age, gender, weight, ethnicity, et cetera); presence/absence of chronic behavioural conditions (smoking, heavy alcohol consumption, consumption of various recreational drugs, et cetera); presence/absence of various chronic clinical conditions (high blood pressure, diabetes, asthma, heart disease; et cetera); presence/absence of various acute ailments (pneumonia or other acute respiratory ailments, various oncological conditions, or so forth); features related to same (e.g., cancer stage and grade); medical and care provider information and so forth. Thus, the at least one attribute value are data fields in a medical record, which can represent medical issues, for example, a symptom (e.g. bleeding), a severe medical result (e.g. death) or a medical status (e.g. normal), or other contextual information, for example, medical histories, lab test results or demographic data of a patient. The attribute value may be the value of contextual information or the likelihood of a medical issue for example.

In an example, the at least one data source comprises one or more clinical data repositories (CDR), lab information system (LIS), and admission notes (AN).

In this manner, the noise inherited including incompleteness and multiple potentially conflicting sources of data can be mitigated providing an efficient and effective means to help users understand and make optimized decisions on what hypotheses (models) to validate considering the status (availability) of CDR data for example.

According to an example, the input module is configured to enable a user to provide constraints of expected data. The data and model analysis module is configured to determine for each model of the plurality of models statistics of variables specified in the constraints of expected data provided by the user. The selection of the sub-set of models then comprises utilization of the determined statistics of variables for the plurality of models.

In an example, the statistics of variables specified in the user constraints comprises for example record count, gender, age in expected record number, gender ratios, age ranges.

According to an example, the optimization module is configured to output information on whether any data records of the available data records for the top models are outside of the constraints of expected data input by the user.

According to an example, the data and model analysis module is configured to determine at least one model performance for at least one model of the plurality of models. The selection of the sub-set of models then comprises utilization of the at least one model performance.

According to an example, the optimization module is configured to determine a best model of the sub-set of models.

According to an example, the optimization module is configured to rank the sub-set of models.

According to an example, the data and model analysis module is configured to determine a number of clean data records of the available data records for each model. Each of the available data records can comprise a plurality of values, and the clean data records can be determined as those data records of the available data records that are missing less than a threshold number of values. The availability measure for a model then comprises the number of clean data records for that model.

According to an example, the availability measure for the model comprises the number of clean data records for that model divided by the total number of clean data records.

According to an example, the threshold value is a percentage of the number of values, which comprises zero percent.

Thus for example, clean data records can be those data records that have no missing values, have less than 1% of missing values, have less than 2% of missing values, have less than 5% of missing values, have less than 10% of missing values, have less than 20% of missing values.

According to an example, the availability measure for a model has a value that is weighted by the number of model variables for that model.

In an example, the at least one data source comprises CDR, LIS and admission noted (AN).

According to an example, the data and model analysis module is configured to derive at least one model variable for at least one of the plurality of models. The derivation comprises matching at least some of the model variables input by the user with corresponding at least one source variable.

According to an example, the input module is configured to enable the user to input a search query. The user specifying the plurality of models can then comprise the input unit identifying at least one model according to the search query.

Fig. 2 shows an example of a system 100 for patient data availability analysis. The system 100 comprises at least one data source 110, and an apparatus 10 for patient data availability analysis as described with respect to Fig. 2. The system 100 also comprises an output unit 120. The plurality patient records are provided from the at least one data source 110 to the input unit 20. The output unit 120 is configured to output information relating to the top models of data availability.

Fig. 3 shows a method 200 for patient data availability analysis in its basic steps. The method 200 comprises:
in a specifying step 210, also referred to as step a), specifying a plurality of models, wherein each model provides output as a function of model variables;
in a defining step 220, also referred to as step b), defining at least some model variables for the plurality of models;
in a specifying step 230, also referred to as step c), specifying source variables, wherein a model variable can be derived from one or more source variables;
in a specifying step 240, also referred to as step d), specifying at least one data source;
in a receiving step 250, also referred to as step e), receiving a plurality of data records from the at least one data source, wherein each data record comprises at least one attribute;
in a determining step 260, also referred to as step f), determining a set of available data records for each model from the plurality of data records, the determination for a model comprising utilizing the model variables for that model, the associated source variables and the at least one attribute for the plurality of data records;
in a determining step 270, also referred to as step g), determining a plurality of availability measures for the corresponding plurality of models, the determination comprising utilizing the determined set of available data records for each model; and
in a selecting step 280, also referred to as step h), selecting a sub-set of models of the plurality of models as top models of data availability, wherein the selection comprises utilizing the plurality of availability measures.

In an example, wherein the method comprises providing constraints of expected data, and comprises determining for the top models statistics of variables specified in the constraints of expected data. The selection of the sub-set of models then comprises utilization of the determined statistics of variables.

In an example, the method comprises outputting information on whether any data records of the available data records for a model are outside of (do not satisfy) the constraints of expected data.

In an example, the method comprises determining at least one model performance for at least one model of the plurality of models. The selecting the sub-set of models then comprises utilizing the at least one model performance.

In an example, the method comprises determining a best model of the sub-set of models.

In an example, the method comprises ranking the sub-set of models.

In an example, the method comprises determining a number of clean data records of the available data records for each model, where each of the available data records comprises a plurality of values. The clean data records are then determined as those data records of the available data records that are missing less than a threshold number of values. The availability measure for a model then comprises the number of clean data records for that model.

In an example, determining the availability measure for the model comprises calculating the number of clean data records for that model divided by the total number of clean data records.

In an example of the method, the threshold value is a percentage of the number of values, which comprises zero percent.

In an example of the method, the availability measure for a model has a value that is weighted by the number of model variables for that model.

In an example, the method comprises deriving at least one model variable for at least one of the plurality of models, the derivation comprising matching at least some of the model variables with corresponding at least one source variable.

In an example, the method comprises inputting a search query, and wherein specifying the plurality of models comprise identifying at least one model according to the search query.

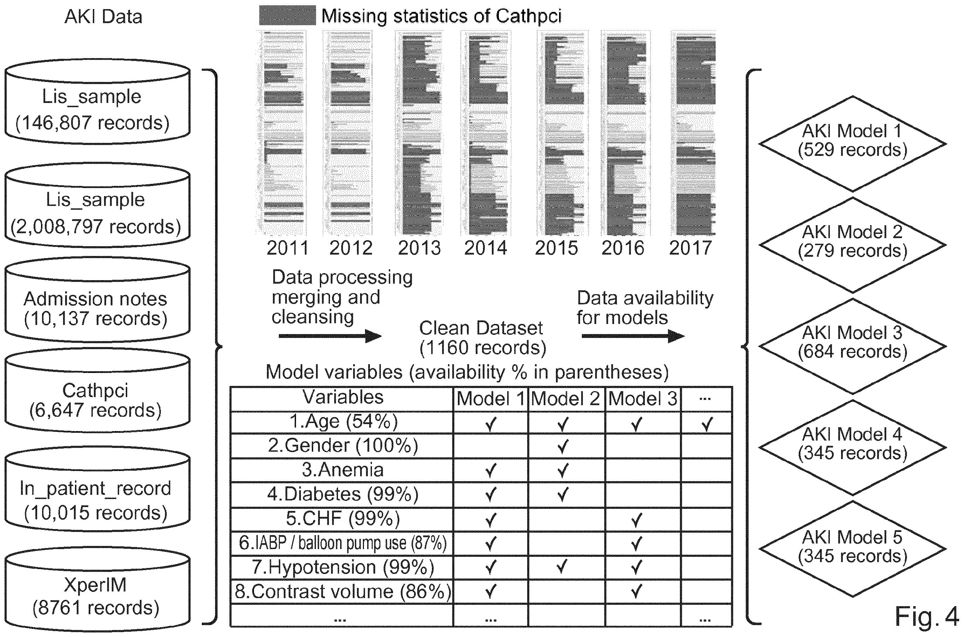

The apparatus, system and method for patient data availability analysis are now described in more detail in conjunction with Figs. 4-5.

Fig. 4 shows a detailed high-level view of a system for patient data availability analysis relating to the example of Acute Kidney Injury (AKI). As discussed above, Cardiovascular Information Systems (CVIS) have been available for many years in the healthcare industry. The main function of current CVIS is to store and assess patients' record - see for example US6604115B1. Some sophisticated CVIS are also integrated with other systems in the hospital such as Electronic Health Record (EHR), Laboratory Information System (LIS), etc. As such, a physician will have an overview of patients' health record. Usually a CVIS is used for reporting, scheduling and management purposes, whilst a Clinical Data Repository (CDR) further records more in-depth information for performing advanced data analytics such as modeling and predictive analytics. Here, CVIS and CDR are collectively termed CDR.

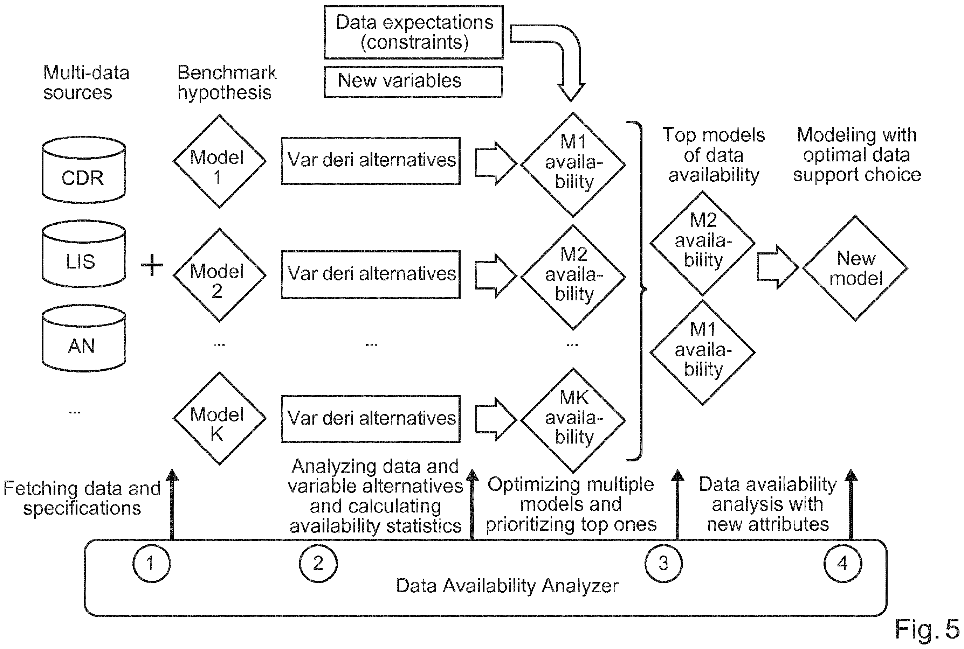

The apparatus, method and system for patient record identification as described with respect to Figs 1-3, embodied in the exemplar system shown in Fig. 4 and described below with respect to the detailed workflow shown in Fig. 5 provides a directly integrated module in the CDR to allow users/physicians to turn the information stored into knowledge effective and efficiently.

Typically a (clinical) research topic centers on validating a hypothesis (here also called a model), or shortlisting from relevant hypotheses, from data collected (or to be collected). Therefore, data availability is critical for whether the hypotheses can be validated before real prediction analysis and risk modeling can be done. In traditional designed studies, data was collected in a very strict manner, with dedicated efforts on quality, stringent inclusion /exclusion criteria and human intensive follow-ups. This way of working restricts the scalability and requires huge cost and labor for population-wide representative conclusions. On the other hand, with the widely adopted electronic records and health information systems, as well as in-depth CDR, hypotheses can be generated and validated using the tremendous existing data collected day-after-day. The challenge lies on the noise inherited including incompleteness and multiple potentially conflicting sources of data. The system of apparatus, system and method embodied in the detailed examples shown in figs 4-5 by providing an efficient and effective tool to help users understand and make optimized decisions on what hypotheses (models) to validate considering the status (availability) of CDR data.

Fig. 4 shows a system that utilizes CDR data and data from other systems to build Acute Kidney Injury (AKI) prediction models. There are several existing AKI risk scores or hypotheses (models 1 to 5) which were derived by logistic regression from patient data. To validate whether the existing risk scores are predictive for specific data and to further build population specific models (e.g. logistic regression) with more informative variables, it is first needed to clearly understand how much data can support existing and new models. As shown in Fig. 4, the CDR as well as the other data sources have missing values, and can provide different availability for the various variables of the models. The variables for the models are here shown as: Age, Gender; Anemia; Diabetes; CHF; IABP/balloon pump use; Hypertension; Contrast volume, and other model variables are possible.

There are a number of challenges, at multiple levels, that are addressed:
1. Different models have different variables, and data may support them with significant different availability;
2. The same variable can come from different data sources or can be derived using multiple ways (e.g. combining multiple lab results), and data may support them with significant different availability;
3. Introducing new variables to validate new hypotheses (to build new models) is also subject to constraints of data availability (less clean data for more variables).

Continuing with Fig. 4, and taking the AKI data that is being provided in as an example, there are Lab Information System (LIS) data, admission notes from EMR, CDR (coded as Cathpci) data, as well as other sources. Each data source can have considerable missing values due to various reasons as discussed above. Also, different models (see models 1-5) result on different data availability (ranging from 279 records to 684 records). This determination of available data records enables the next steps of prediction and modeling to be carried out efficiently.

To help understand the issues being addressed by the described apparatus, system and method for patient data availability analysis consider the following: Anemia (Yes/No) may be available directly from CDR with considerable missing values, and may also be derived by checking LIS if baseline hemoglobin < 10g/dL (100g/L). However, different combinations of ways to derive single variables, multiplied by the various variables that require derivations, result in a non-trivial number of data availability combinations that users cannot possibly directly handle. For simplicity, it is assumed the models all have the same variable requirements. Imagine there are 5 models, each model has 8 variables, 2 out of 8 variables have each 4 total different ways of derivations, and the other 3 have each 2 possible sources. The remaining 3 variables are from the same source with no need of derivation. There are therefore 5 x (42 x23 x13) = 640 combinations. Adding each additional variable to build a new model from these choices leads to another level of multiplication (*2 each time if a new variable has two alternative ways). Furthermore, for a certain clinical relevant research study, the user (a clinician) would expect some requirements to be met, such as minimal patient numbers, gender ratios, age ranges, etc. This further complicates the feasibility and makes it unclear for the user to be confident to reach his study objectives: if hypotheses can be rigorously validated, or it fails to reach any conclusions simply due to unavailability of data.

The system of apparatus, system and method embodied in the detailed examples shown in Figs 4-5, addresses this by analyzing data availability during shortlisting among multiple hypotheses (models) prior to further prediction and modeling steps. Thus, as shown on the right hand side of Fig. 4, it is desirable to have data availability selection at the first level to determine the best availability among different variable source and derivation choices to simplify decisions on follow-up analysis, otherwise each model would have non-trivial combinations to be selected. And it is desirable (not shown in Fig. 4) to provide to the user the best available models according to data (optionally with also prediction power considering available data). In this manner a data processing system is provided that trades off data availability trade for the selection of different hypotheses (e.g. models for predictive analytics) on noisy data. In other words, the data availability analysis system for clinical data repositories (CDR) as described here enables self-service of clinical research on noisy practice data. For a typical clinical research problem, a number of input variables (features) are needed to be extracted from a CDR and there can be multiple ways to obtain these variables from different sources through different rules, yet resulting in different data availability due to noises in the data. With the system, users can conveniently understand the trade-off between desired input variables and the data size that supports the set of desired variables. They can also identify potential data quality issues by variable-availability profiling, and check if the data supports their study hypothesis (model) to be further validated by follow-up data analytics.

Fig. 5 shows a detailed workflow relating to a system for patient data availability analysis, consisting of the following main elements as described below with associated functionalities operating within the workflow:
1. An input module for users to specify data sources, models, model variables, and data expectations (constraints), collected denoted as specifications.
   - The system provides interfaces for users to input variables of models, specify source variables for variable derivation, and provide constraints of the expected data.
   - It retrieves existing risk models according to user search or key words, from knowledge bases in the clinical domain, and thus simplifies the input of model variable and variable derivation rules. However, it is not necessary for risk models to be retrieved in this way.
2. A data and hypothesis (model) analysis module to consolidate multiple data sources and specifications for data availability statistics.
   - The system maintains a list of related data sources such as CDR, LIS and admission notes (AN).
   - It matches the hypothesis input variables, corresponding source variables to derive hypothesis variables, target variables - thus data is retrieved after specifying the model variables and target variables.
   - For a specific model, its variables and source variables, and the corresponding data attributes from multiple data sources, the system retrieves the available data and computes the following:
      i. A availability measure, of which an embodiment can be the number of available records;
      ii. The statistics of variables specified in the user constraints (e.g. record count, gender, age in expected record number, gender ratios, age ranges); and
      iii. The model performance, if the hypothesis is a model which can be evaluated, e.g. on area-under-curve (AUC) on a receiver operating characteristics curve (ROC). Computation of the model performance can aid in determining patient data availability, but is not necessary.
   - It relies on a connected self-service data cleansing system to unify aliases of equivalent attributes, merging conflicts of different data sources, detecting and removing noisy data.
3. An optimization module to optimize availability measures considering data availability and user data expectations (constraints).
   - The system works on the resultant statistics (availability measure, variable statistics of user constraints, and optionally model performance) and optimizes an overall availability score out of the varying variable alternatives.
   - The system shortlists and presents to the user the top models according to a single overall score or a multi-objective pareto-front.
   - The system outputs the information of compromised user expectations (violated constraints)
4. A new attribute analysis module to accommodate data availability analysis for user interested new attributes , and as such is in effect an "add-on" to the above described workflow.
   - The system further supports introduction of new variables of interest according to user need for more information on advanced analytics.
   - The system performs similar steps and gives new model data availability results.
   - Thus the new attribute analysis module addresses a scenario where the users would like to add more attributes (model variables) later on.

More detail is provided on several of the features described above:
1. The "availability measure" described at point 2:
   - In one baseline example, the count of records with no missing values (a pure clean set) for a specific model's specific variable alternative choice can be used as the availability measure, optionally normalized by the total count of records with no missing in the target variable.
   - In another example, the above normalized count can be further weighed by accepting certain missing percentages of the records, introducing a flexible control when available data cannot be purely clean.
   - In another example, the measure can be further weighed by the number of variables where a more descriptive model (more variables) with sufficient available data is favored.
2. The "optimization module" described at point 3, that optimizes all combinations of model and variable alternatives:
   - In one baseline example, all combinations can be exhaustively assessed according to the optimization measure (a baseline can be the availability measure, as described above).
   - In another example, a stochastic search algorithm such as random search, genetic algorithm, can be employed if the number of combinations is intractably large.
   - all user specified data expectations (availability constraints) can be set as hard constraints and any combination that does not meet the constraints will not be proceeded for optimization.
   - In another example, the user specified availability constraints can be merged as part of a new availability measure, or added as an additional penalty term. Then the availability optimization results can be such that they compromise (violate) certain user specified expectations, e.g. gender ratio is 1:3 while the expected ratio was 1:4. The discrepancy, in terms of such a violation of user specified expectations, can be presented to user for user selection and to help guide decision making.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus (10) for patient data availability analysis, comprising:
- an input module (20);
- a data and model analysis module (30); and
- an optimization module (40);
wherein, the input module is configured to enable a user to specify a plurality of models, wherein each model being used in clinical research and predicting a disease outcome, wherein each model provides output as a function of model variables, and the input module is configured to enable the user to define at least some model variables for the plurality of models, and the input module is configured to enable the user to specify source variables, wherein a model variable can be derived from one or more source variables, and the input module is configured to enable the user to specify at least one data source;
wherein, the input module is configured to receive a plurality of data records from the at least one data source, wherein each data record comprises at least one attribute;
wherein, the data and model analysis module is configured to determine a set of available data records for each model from the plurality of data records, the determination for a model comprising utilization of the model variables for that model, the associated source variables and the at least one attribute for the plurality of data records;
wherein the data and model analysis module is configured to determine a number of clean data records of the available data records for each model, wherein each of the available data records comprises a plurality of values, and wherein the clean data records are determined as those data records of the available data records that are missing less than a threshold number of values;
wherein, the data and model analysis module is configured to determine a plurality of availability measures for the corresponding plurality of models, wherein the determination comprises utilization of the determined set of available data records for each model, and wherein the availability measure for a model comprises the number of clean data records for that model; and
wherein, the optimization module is configured to rank and select a sub-set of models of the plurality of models as top models of data availability, wherein the selection comprises utilization of the plurality of availability measures.

2. Apparatus according to claim 1, wherein, the input module is configured to enable a user to provide constraints of expected data, and wherein the data and model analysis module is configured to determine for each model of the plurality of models statistics of variables specified in the constraints of expected data provided by the user, and wherein the selection of the sub-set of models comprises utilization of the determined statistics of variables for the plurality of models.

3. Apparatus according to claim 2, wherein the optimization module is configured to output information on whether any data records of the available data records for the top models are outside of the constraints of expected data input by the user.

4. Apparatus according to any of claims 1-3, wherein the data and model analysis module is configured to determine at least one model performance for at least one model of the plurality of models, and wherein the selection of the sub-set of models comprises utilization of the at least one model performance.

5. Apparatus according to any of claims 1-4, wherein the optimization module is configured to determine a best model of the sub-set of models.

6. Apparatus according to any of claims 1-4, wherein the optimization module is configured to rank the sub-set of models.

7. Apparatus according to any of claims 1-6, wherein the availability measure for the model comprises the number of clean data records for that model divided by the total number of clean data records.

8. Apparatus according to any of claims 1-7, wherein the threshold value is a percentage of the number of values, which comprises zero percent.

9. Apparatus according to any of claims 1-8, wherein the availability measure for a model has a value that is weighted by the number of model variables for that model.

10. Apparatus according to any of claims 1-9, wherein the data and model analysis module is configured to derive at least one model variable for at least one of the plurality of models, the derivation comprising matching at least some of the model variables input by the user with corresponding at least one source variable.

11. Apparatus according to any of claims 1-10, wherein the input module is configured to enable the user to input a search query, and wherein the user specifying the plurality of models comprises the input unit identifying at least one model according to the search query.

12. A system (100) for patient data availability analysis, comprising:
- at least one data source (110);
- an apparatus (10) for patient data availability analysis according to any of the preceding claims; and
- an output unit (120);
wherein, the plurality patient records are provided from the at least one data source to the input unit (20); and
wherein, the output unit is configured to output information relating to the top models of data availability.

13. A computer-implemented method (200) for patient data availability analysis, comprising
a) specifying (210) a plurality of models, wherein each model being used in clinical research and predicting a disease outcome, wherein each model provides output as a function of model variables;
b) defining (220) at least some model variables for the plurality of models;
c) specifying (230) source variables, wherein a model variable can be derived from one or more source variables;
d) specifying (240) at least one data source;
e) receiving (250) a plurality of data records from the at least one data source, wherein each data record comprises at least one attribute;
f) determining (260) a set of available data records for each model from the plurality of data records, the determination for a model comprising utilizing the model variables for that model, the associated source variables and the at least one attribute for the plurality of data records, and determining a number of clean data records of the available data records for each model, wherein each of the available data records comprises a plurality of values, and wherein the clean data records are determined as those data records of the available data records that are missing less than a threshold number of values;
g) determining (270) a plurality of availability measures for the corresponding plurality of models, wherein the determination comprises utilizing the determined set of available data records for each model, and wherein the availability measure for a model comprises the number of clean data records for that model; and
h) selecting and ranking and (280) a sub-set of models of the plurality of models as top models of data availability, wherein the selection comprises utilizing the plurality of availability measures.

14. A computer program element for controlling an apparatus according to one of claims 1 to 11 and/or a system according to claim 12, which when executed by a processor is configured to carry out the method of claim 13.

## Patentansprüche

1. Vorrichtung (10) zur Analyse der Patientendatenverfügbarkeit, umfassend:
- ein Eingabemodul (20);
- ein Daten- und Modellanalysemodul (30); und
- ein Optimierungsmodul (40);
wobei das Eingabemodul so konfiguriert ist, dass es einem Benutzer ermöglicht, eine Vielzahl von Modellen anzugeben, wobei jedes Modell in der klinischen Forschung verwendet wird und einen Krankheitsverlauf vorhersagt, wobei jedes Modell eine Ausgabe als Funktion von Modellvariablen bereitstellt, und das Eingabemodul ist so konfiguriert, dass es dem Benutzer ermöglicht, mindestens einige Modellvariablen für die Vielzahl von Modellen zu definieren, und das Eingabemodul ist so konfiguriert, dass es dem Benutzer ermöglicht, Quellvariablen anzugeben, wobei eine Modellvariable aus einer oder mehreren Quellvariablen abgeleitet werden kann und das Eingabemodul so konfiguriert ist, dass es dem Benutzer ermöglicht, mindestens eine Datenquelle anzugeben;
wobei das Eingabemodul so konfiguriert ist, dass es mehrere Datensätze von der mindestens einen Datenquelle empfängt, wobei jeder Datensatz mindestens ein Attribut umfasst;
wobei das Daten- und Modellanalysemodul so konfiguriert ist, dass es einen Satz verfügbarer Datensätze für jedes Modell aus der Vielzahl von Datensätzen ermittelt, wobei die Bestimmung für ein Modell die Nutzung der Modellvariablen für dieses Modell, der zugehörigen Quellvariablen und des mindestens einen Attributs für die mehreren Datensätze umfasst;
wobei das Daten- und Modellanalysemodul so konfiguriert ist, dass es eine Anzahl sauberer Datensätze der verfügbaren Datensätze für jedes Modell ermittelt, wobei jeder der verfügbaren Datensätze eine Vielzahl von Werten umfasst und wobei die sauberen Datensätze als diejenigen Datensätze der verfügbaren Datensätze bestimmt werden, denen weniger als eine Schwellenwertanzahl an Werten fehlt;
wobei das Daten- und Modellanalysemodul so konfiguriert ist, dass es mehrere Verfügbarkeitsmaße für die entsprechende Vielzahl von Modellen ermittelt, wobei die Bestimmung die Nutzung des ermittelten Satzes verfügbarer Datensätze für jedes Modell umfasst und wobei das Verfügbarkeitsmaß für ein Modell die Anzahl sauberer Datensätze für dieses Modell umfasst; und
wobei das Optimierungsmodul so konfiguriert ist, dass es eine Untergruppe von Modellen aus der Vielzahl von Modellen als Topmodelle der Datenverfügbarkeit einstuft und auswählt, wobei die Auswahl die Nutzung der Vielzahl von Verfügbarkeitsmaßen umfasst.

2. Vorrichtung nach Anspruch 1, wobei das Eingabemodul so konfiguriert ist, dass es einem Benutzer ermöglicht, Einschränkungen erwarteter Daten bereitzustellen, und wobei das Daten- und Modellanalysemodul so konfiguriert ist, dass es für jedes Modell der Vielzahl von Modellen Statistiken von Variablen ermittelt, die in den vom Benutzer bereitgestellten Einschränkungen der erwarteten Daten spezifiziert sind, und wobei die Auswahl der Teilmenge von Modellen die Nutzung der ermittelten Variablenstatistiken für die Vielzahl von Modellen umfasst.

3. Vorrichtung nach Anspruch 2, wobei das Optimierungsmodul dazu konfiguriert ist, Informationen darüber auszugeben, ob Datensätze der verfügbaren Datensätze für die Topmodelle außerhalb der Einschränkungen der erwarteten Dateneingabe durch den Benutzer liegen.

4. Vorrichtung nach einem der Ansprüche 1 bis 3, wobei das Daten- und Modellanalysemodul dazu konfiguriert ist, mindestens eine Modellleistung für mindestens ein Modell aus der Vielzahl von Modellen zu bestimmen, und wobei die Auswahl der Teilmenge von Modellen die Nutzung der mindestens einen Modellleistung umfasst.

5. Vorrichtung nach einem der Ansprüche 1 bis 4, wobei das Optimierungsmodul so konfiguriert ist, dass es ein bestes Modell aus der Teilmenge von Modellen ermittelt.

6. Vorrichtung nach einem der Ansprüche 1 bis 4, wobei das Optimierungsmodul so konfiguriert ist, dass es die Teilmenge der Modelle einstuft.

7. Vorrichtung nach einem der Ansprüche 1 bis 6, wobei das Verfügbarkeitsmaß für das Modell die Anzahl sauberer Datensätze für dieses Modell geteilt durch die Gesamtzahl sauberer Datensätze umfasst.

8. Vorrichtung nach einem der Ansprüche 1 bis 7, wobei der Schwellenwert ein Prozentsatz der Anzahl von Werten ist, der null Prozent umfasst.

9. Vorrichtung nach einem der Ansprüche 1 bis 8, wobei das Verfügbarkeitsmaß für ein Modell einen Wert aufweist, der mit der Anzahl der Modellvariablen für dieses Modell gewichtet wird.

10. Vorrichtung nach einem der Ansprüche 1 bis 9, wobei das Daten- und Modellanalysemodul so konfiguriert ist, dass es mindestens eine Modellvariable für mindestens eines der mehreren Modelle ableitet, wobei die Ableitung den Abgleich mindestens einiger der vom Benutzer eingegebenen Modellvariablen mit der entsprechenden mindestens einer Quellvariablen umfasst.

11. Vorrichtung nach einem der Ansprüche 1 bis 10, wobei das Eingabemodul so konfiguriert ist, dass es dem Benutzer die Eingabe einer Suchanfrage ermöglicht, und wobei der Benutzer, der die Vielzahl von Modellen spezifiziert, umfasst, dass die Eingabeeinheit mindestens ein Modell gemäß der Suchabfrage identifiziert.

12. System (100) zur Analyse der Patientendatenverfügbarkeit, umfassend:
- mindestens eine Datenquelle (110);
- eine Vorrichtung (10) zur Analyse der Patientendatenverfügbarkeit nach einem der vorhergehenden Ansprüche; und
- eine Ausgabeeinheit (120);
wobei die mehreren Patientendatensätze von der mindestens einen Datenquelle an die Eingabeeinheit (20) bereitgestellt werden; und
wobei die Ausgabeeinheit dazu konfiguriert ist, Informationen bezüglich der Topmodelle der Datenverfügbarkeit auszugeben.

13. Computerimplementiertes Verfahren (200) zur Analyse der Verfügbarkeit von Patientendaten, umfassend
a) Angeben (210) einer Vielzahl von Modellen, wobei jedes Modell in der klinischen Forschung verwendet wird und einen Krankheitsverlauf vorhersagt, wobei jedes Modell eine Ausgabe als Funktion von Modellvariablen liefert;
b) Definieren (220) mindestens einiger Modellvariablen für die mehreren Modelle;
c) Angeben (230) von Quellvariablen, wobei eine Modellvariable aus einer oder mehreren Quellvariablen abgeleitet werden kann;
d) Angeben (240) mindestens einer Datenquelle;
e) Empfangen (250) mehrerer Datensätze von der mindestens einen Datenquelle, wobei jeder Datensatz mindestens ein Attribut umfasst;
f) Bestimmen (260) eines Satzes verfügbarer Datensätze für jedes Modell aus der Vielzahl von Datensätzen, wobei die Bestimmung für ein Modell das Verwenden der Modellvariablen für dieses Modell, der zugehörigen Quellvariablen und des mindestens einen Attributs für die mehreren Datensätze und das Bestimmen einer Anzahl sauberer Datensätze der verfügbaren Datensätze für jedes Modell umfasst, wobei jeder der verfügbaren Datensätze mehrere Werte umfasst und wobei die sauberen Datensätze als diejenigen Datensätze der verfügbaren Datensätze bestimmt werden, denen weniger als eine Schwellenwertanzahl an Werten fehlen;
g) Bestimmen (270) einer Vielzahl von Verfügbarkeitsmaßen für die entsprechende Vielzahl von Modellen, wobei die Bestimmung die Verwendung des ermittelten Satzes verfügbarer Datensätze für jedes Modell umfasst und wobei das Verfügbarkeitsmaß für ein Modell die Anzahl sauberer Datensätze für dieses Modell umfasst; und
h) Auswählen und Einstufen (280) einer Teilmenge von Modellen aus der Vielzahl von Modellen als Spitzenmodelle der Datenverfügbarkeit, wobei die Auswahl die Nutzung der Vielzahl von Verfügbarkeitsmaßen umfasst.

14. Computerprogrammelement zur Steuerung einer Vorrichtung nach einem der Ansprüche 1 bis 11 und/oder eines Systems nach Anspruch 12, das bei Ausführung durch einen Prozessor dazu konfiguriert ist, das Verfahren nach Anspruch 13 auszuführen.

## Revendications

1. Appareil (10) pour l'analyse de la disponibilité des données de patients, comprenant:
- un module d'entrée (20);
- un module d'analyse de données et de modèles (30) ; et
- un module d'optimisation (40);
où, le module d'entrée est configuré pour permettre à un utilisateur de spécifier une pluralité de modèles, où chaque modèle est utilisé dans la recherche clinique et est prédictif de l'évolution d'une maladie, où chaque modèle fournit une sortie en fonction de variables de modèle, et le module d'entrée est configuré pour permettre à l'utilisateur de définir au moins certaines variables de modèle pour la pluralité de modèles, et le module d'entrée est configuré pour permettre à l'utilisateur de spécifier des variables de source, où une variable de modèle peut être dérivée d'une ou plusieurs variables de source, et le module d'entrée est configuré pour permettre à l'utilisateur de spécifier au moins une source de données;
où, le module d'entrée est configuré pour recevoir une pluralité d'enregistrements de données provenant de l'au moins une des sources de données, où chaque enregistrement de données comprend au moins un attribut;
où, le module d'analyse de données et de modèles est configuré pour déterminer un ensemble d'enregistrements de données disponibles pour chaque modèle à partir de la pluralité d'enregistrements de données, la détermination pour un modèle comprenant l'utilisation des variables de modèle pour ce modèle, des variables de source associées et de l'au moins un attribut pour la pluralité d'enregistrements de données;
où, le module d'analyse de données et de modèles est configuré pour déterminer un certain nombre d'enregistrements de données nettoyées parmi les enregistrements de données disponibles pour chaque modèle, où chacun des enregistrements de données disponibles comprend une pluralité de valeurs, et où les enregistrements de données nettoyées sont déterminés comme étant ces enregistrements de données parmi les enregistrements de données disponibles qui manquent moins d'un nombre seuil de valeurs;
où, le module d'analyse de données et de modèles est configuré pour déterminer une pluralité de mesures de disponibilité pour la pluralité correspondante de modèles, où la détermination comprend l'utilisation de l'ensemble déterminé d'enregistrements de données disponibles pour chaque modèle, et où la mesure de disponibilité pour un modèle comprend le nombre d'enregistrements de données nettoyées pour ce modèle; et
où, le module d'optimisation est configuré pour classer et sélectionner un sous-ensemble de modèles parmi la pluralité de modèles en tant que modèles supérieurs de disponibilité de données, où la sélection comprend l'utilisation de la pluralité de mesures de disponibilité.

2. Appareil selon la revendication 1, dans lequel le module d'entrée est configuré pour permettre à un utilisateur de fournir des contraintes de données attendues, et dans lequel le module d'analyse de données et de modèles est configuré pour déterminer pour chaque modèle parmi la pluralité de modèles des statistiques de variables spécifiées dans les contraintes de données attendues fournies par l'utilisateur, et dans lequel la sélection du sous-ensemble de modèles comprend l'utilisation des statistiques déterminées de variables pour la pluralité de modèles.

3. Appareil selon la revendication 2, dans lequel le module d'optimisation est configuré pour fournir des informations indiquant si des enregistrements de données parmi les enregistrements de données disponibles pour les modèles supérieurs sont en dehors des contraintes de données attendues entrées par l'utilisateur.

4. Appareil selon l'une quelconque des revendications 1 à 3, dans lequel le module d'analyse de données et de modèles est configuré pour déterminer au moins une performance de modèle pour au moins un modèle parmi la pluralité de modèles, et dans lequel la sélection du sous-ensemble de modèles comprend l'utilisation de l'au moins une performance de modèle.

5. Appareil selon l'une quelconque des revendications 1 à 4, dans lequel le module d'optimisation est configuré pour déterminer un modèle meilleur du sous-ensemble de modèles.

6. Appareil selon l'une quelconque des revendications 1 à 4, dans lequel le module d'optimisation est configuré pour classer le sous-ensemble de modèles.

7. Appareil selon l'une quelconque des revendications 1 à 6, dans lequel la mesure de disponibilité pour le modèle comprend le nombre d'enregistrements de données nettoyées pour ce modèle divisé par le nombre total d'enregistrements de données nettoyées.

8. Appareil selon l'une quelconque des revendications 1 à 7, dans lequel la valeur seuil est un pourcentage du nombre de valeurs, qui comprend zéro pourcent.

9. Appareil selon l'une quelconque des revendications 1 à 8, dans lequel la mesure de disponibilité pour un modèle a une valeur qui est pondérée par le nombre de variables de modèle pour ce modèle.

10. Appareil selon l'une quelconque des revendications 1 à 9, dans lequel le module d'analyse de données et de modèles est configuré pour dériver au moins une variable de modèle pour au moins un parmi la pluralité de modèles, la dérivation comprenant la mise en correspondance d'au moins certaines des variables de modèle entrées par l'utilisateur avec au moins une variable de source correspondante.

11. Appareil selon l'une quelconque des revendications 1 à 10, dans lequel le module d'entrée est configuré pour permettre à l'utilisateur d'entrer une requête de recherche, et dans lequel l'utilisateur spécifiant la pluralité de modèles comprend l'unité d'entrée identifiant au moins un modèle selon la requête de recherche.

12. Système (100) d'analyse de la disponibilité des données de patients, comprenant:
- au moins une source de données (110);
- un appareil (10) pour l'analyse de la disponibilité des données de patients selon l'une quelconque des revendications précédentes; et
- une unité de sortie (120);
où, la pluralité d'enregistrements de patients est fournie depuis l'au moins une source de données vers l'unité d'entrée (20); et
où, l'unité de sortie est configurée pour fournir des informations relatives aux modèles supérieurs de disponibilité de données.

13. Procédé mis en œuvre par ordinateur (200) pour l'analyse de la disponibilité des données de patients, consistant à
a) spécifier (210) une pluralité de modèles, où chaque modèle est utilisé dans la recherche clinique et est prédictif de l'évolution d'une maladie, où chaque modèle fournit une sortie en fonction de variables de modèle;
b) définir (220) au moins certaines variables de modèle pour la pluralité de modèles;
c) spécifier (230) des variables de source, où une variable de modèle peut être dérivée d'une ou plusieurs variables de source;
d) spécifier (240) au moins une source de données;
e) recevoir (250) une pluralité d'enregistrements de données provenant de l'au moins une source de données, où chaque enregistrement de données comprend au moins un attribut;
f) déterminer (260) un ensemble d'enregistrements de données disponibles pour chaque modèle à partir de la pluralité d'enregistrements de données, la détermination pour un modèle comprenant l'utilisation des variables de modèle pour ce modèle, des variables de source associées et de l'au moins un attribut pour la pluralité d'enregistrements de données, et déterminer un nombre d'enregistrements de données nettoyées parmi les enregistrements de données disponibles pour chaque modèle, où chacun des enregistrements de données disponibles comprend une pluralité de valeurs, et où les enregistrements de données nettoyées sont déterminés comme étant les enregistrements de données des enregistrements de données disponibles qui manquent moins d'un nombre seuil de valeurs;
g) déterminer (270) une pluralité de mesures de disponibilité pour la pluralité correspondante de modèles, où la détermination comprend l'utilisation de l'ensemble déterminé de enregistrements de données disponibles pour chaque modèle, et où la mesure de disponibilité pour un modèle comprend le nombre d'enregistrements de données nettoyées pour ce modèle; et
h) sélectionner et classer (280) un sous-ensemble de modèles parmi la pluralité de modèles en tant que modèles supérieurs de disponibilité de données, où la sélection comprend l'utilisation de la pluralité de mesures de disponibilité.

14. Elément de programme informatique pour commander un appareil selon l'une des revendications 1 à 11 et/ou un système selon la revendication 12, qui lorsqu'il est exécuté par un processeur est configuré pour mettre en œuvre le procédé selon la revendication 13.
